# EUROPEAN PATENT APPLICATION

(11) **EP 4 141 118 A1**
(43) Date of publication of application: **01.03.2023**
(21) Application number: 21792496.8
(22) Date of filing: 14.04.2021
(51) Int. Cl.: C12N 15/62, C12N 5/10, C12N 15/867, C07K 19/00

(54) **TECHNIQUE FOR PREPARING UNIVERSAL HUMANISED CAR19-DNT CELLS AND APPLICATION THEREFOR**

(30) Priority: 20.04.2020 CN 202010314336
(71) Applicant: Zhejiang Ruijiamei Biotech Co., Ltd., Shaoxing, Zhejiang 312000 (CN)
(72) Inventor: YANG, Liming, Shaoxing, Zhejiang 312000 (CN); WANG, Dan, Shaoxing, Zhejiang 312000 (CN); LI, Xiancai, Shaoxing, Zhejiang 312000 (CN)
(74) Representative: Cabinet Laurent & Charras
(86) International application number: PCT/CN2021/087311
(87) International publication number: WO 2021/213235

(57) **Abstract**

Provided are a technique for preparing universal humanised CAR19-DNT cells and an application therefor. Specifically provided are universal CAR-T cells targeting CD19, the universal CAR-T cells expressing an exogenous CAR construct, and the CAR construct having the structure shown in formula I: L-scFv-H-TM-C-CD3ζ (formula I); in the formula, L is nothing or a signal peptide sequence; scFv is an antibody single-chain fragment variable region sequence targeting CD19; H is nothing or a hinge area; TM is a transmembrane domain; C is a costimulatory signal molecule; and CD3ζ is a cytoplasmic signal conduction sequence derived from CD3ζ. The provided universal humanised CAR19-DNT cells have the advantages of low immunogenicity, not requiring gene editing to avoid GvHD, high safety, high specificity, and obvious tumour killing effects, and the provided construction method can be implemented in large-scale production, the production costs being low.

## Description

### Technical field

The present invention belongs to the technical field of tumor immune cell therapy, and specifically relates to a technical method for preparing universal humanized antigen chimeric receptor CART-19 cells and its application.

### Background

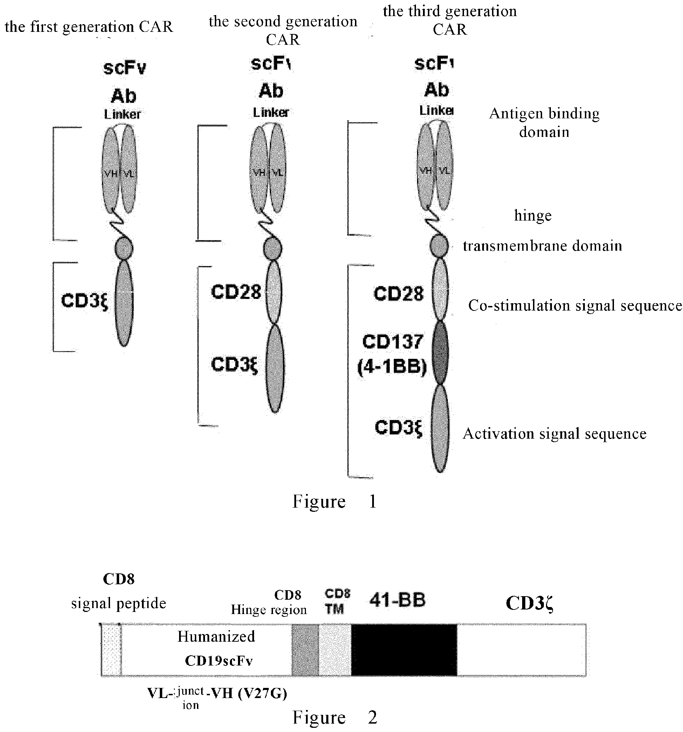

Chimeric Antigen Receptor T (CART) cells are one of the most promising tumor immunotherapies. The two CART cell products that have been approved by the FDA in US in 2017 are both isolated from tumor patients' own Peripheral Blood Mononuclear Cell (PBMC) and genetically engineered to embed specific antigen receptors (CARs), resulting in enhanced targeting, killing activity and persistence of T cells in PBMCs, the recognition of tumor cell surface antigens is not dependent on the restriction of MHC. A common CAR consists of an extracellular antigen-binding region, a transmembrane region, and an intracellular signal transduction region for T cell receptors (e.g. CD3ζ and CD28). The extracellular antigen-binding region consists of the light chain (VL) and heavy chain (VH) of monoclonal antibodies, joined by a hinge in the middle to form a single-chain antibody (single chain fragment variable, scFv), which is capable of recognizing specific tumor antigens. CAR-T cell technology has evolved to the third generation, with the first generation CAR consisting of a single-chain antibody (scFv) antigen-binding site that recognizes a tumor surface antigen and an immunoreceptor tyrosine-activated motif and the extracellular recognition of tumor marker molecules by single-chain antibody (scFv), but there is no co-stimulatory signal, so the clinical performance is poor, which is characterized by short retention time of T cells in vivo and low cytokine secretion capacity. The second generation CART technology introduced co-stimulatory signaling sequence, which can improve the cytotoxic activity, proliferation ability and in vivo survival time of T cells and promote the release of cytokines, and achieved success in the clinical treatment of acute lymphoblastic leukemia; the third generation CART, which arranged two co-stimulatory molecules, CD28 and 4-1BB, and CD3ζ molecules in tandem in the cytoplasmic region, the signal sequence of the double co-stimulation has better results. The structure schematic of the third generation CART is shown in Figure 1.

Currently, autologous CART cells are used to treat B Acute Lymphoblastic cell Leukemia (B-ALL) targeting CD19, achieving complete remission rates of up to 90% or more; in the treatment of lymphoma, complete remission rates are also up to 64%; CART technology has achieved results in the treatment of these two diseases that are unmatched by any other drug. In China, acute lymphocytic leukemia accounts for 80% of acute leukemia, with an incidence rate of 1 in 100,000, and accounts for 20% of all adult leukemia incidences. Immune cell therapy is an important treatment for many serious diseases, including cancer, autoimmune diseases and even serious viral infections have been successfully treated with immune cell therapy.

Although targeting CD19 CART has been highly effective in clinical trials, it also has many drawbacks, besides causing serious side effects such as cytokine storm, there are four major problems: first, some advanced patients with low numbers or poor quality lymphocytes are lost to CART treatment because they do not have sufficient numbers of autoimmune cells; second, the use of CART products prepared from patients' autologous cells has been reported to result in the death of the patient by transduction of viral genes into residual peripheral blood tumor cells; furthermore, most of the universal CART cell products currently under development are designed to knock out genes that can cause graft-versus-host disease, such as the TCR receptor, by gene editing techniques, but knocking out these genes requires a tedious construction process and large-scale sequencing, and has a high off-target rate; finally, since the preparation of autologous CART cell products is a 1-to-1 individualized therapy, the preparation cost is expensive and the preparation process requirements are complex, resulting in expensive products that are unaffordable for most patients and increase the social medical burden.

Therefore, there is an urgent need in the field to develop a universal (ready-to-use) CART cell and its construction method that is derived from the peripheral blood of healthy donors, does not require complex gene editing methods to knock out the TCR gene that causes graft-versus-host disease, has low immunogenicity (does not cause host-versus-graft immune response and has a long in vivo retention time), has high safety, has significant killing effect, and has low production cost.

### Summary of the invention

In view of the series of problems of the existing related technology, the present invention provides a technical method for preparing universal antigen chimeric receptor CAR19-DNT cells and its application.

The purpose of the present invention is to provide a universal (ready-to-use) CART cell and its construction method derived from the peripheral blood of a healthy donor, which does not require complex gene editing methods to knock out the TCR gene that causes graft-versus-host disease, has low immunogenicity (does not cause host-versus-graft immune response and has a long in vivo retention time), has high safety, has a significant killing effect, and has a low production cost.

In a first aspect of the present invention, it provides a universal CAR-T cell targeting CD19, the universal CAR-T cell expressing an exogenous CAR construct, the CAR construct having a structure as shown in Formula I,

L-scFv-H-TM-C-CD3ζ (Formula I)

wherein
L is none or a signal peptide sequence;
scFv is a sequence of the single-chain variable region of an antibody targeting CD 19;
H is none or a hinge region;
TM is a transmembrane domain;
C is a co-stimulatory signaling molecule;
CD3ζ is a cytoplasmic signaling sequence derived from CD3ζ;
esach "-" independently indicates a linking peptide or peptide bond connecting each of the above elements.

In another preferred example, the universal CAR-T cell targeting CD19 is a universal CAR-double negative T cell targeting CD19 (CAR-DNT cell).

In another preferred embodiment, the TCR in the universal CAR-T cell is knocked out or not knocked out.

In another preferred embodiment, the TCR in the universal CAR-T cell is not knocked out.

In another preferred example, the L is a signaling peptide selected from the group consisting of the following protein: CD8, GM-CSF, CD4, CD137, and a combination thereof.

In another preferred embodiment, the L is a CD8-derived signal peptide.

In another preferred example, the scFv comprises a heavy chain variable region (VH) having an amino acid sequence as shown in SEQ ID NO: 6 and a light chain variable region (VL) having an amino acid sequence as shown in SEQ ID NO: 5.

In another preferred example, the scFv further comprises a linking peptide sequence between VH and VL.

In another preferred example, the linking peptide sequence has an amino acid sequence as shown in SEQ ID NO: 7.

In a further preferred embodiment, the scFv has an amino acid sequence as shown in SEQ ID NO: 4.

In another preferred embodiment, the H is a hinge region selected from the group consisting of the following protein: CD8, CD28, CD137, and a combination thereof.

In another preferred embodiment, the H is a CD8-derived hinge region.

In another preferred example, the TM is a transmembrane region of a protein selected from the group consisting of: CD28, CD3 epsilon, CD45, CD4, CD5, CD8, CD9, CD16, CD22, CD33, CD37, CD64, CD80, CD86, CD134, CD137, CD154, and a combination thereof.

In another preferred example, the TM comprises a CD8-derived transmembrane region, and/or a CD28-derived transmembrane region.

In another preferred example, the C is a co-stimulatory signaling molecule of a protein selected from the group consisting of: OX40, CD2, CD7, CD27, CD28, CD30, CD40, CD70, CD134, 4-1BB (CD137), PD1, Dap10, CDS, ICAM-1, LFA-1 (CD11a/CD18), ICOS ( CD278), NKG2D, GITR, TLR2, and a combination thereof.

In another preferred example, C comprises a 4-1BB-derived co-stimulatory signaling molecule, and/or a CD28-derived co-stimulatory signaling molecule.

In another preferred example, the CAR construct has an amino acid sequence as shown in SEQ ID NO: 14.

In a second aspect of the present invention, it provides a method for preparing a universal CAR-DNT cell targeting CD19 as described in a first aspect of the present invention, comprising the steps of:
(i) providing an expression vector, the expression vector containing a nucleotide sequence encoding a CAR construct as shown in Formula I;

   L-scFv-H-TM-C-CD3ζ (Formula I)

   wherein
   L is none or a signal peptide sequence;
   scFv is a sequence of the single-chain variable region of an antibody targeting CD 19;
   H is none or a hinge region;
   TM is a transmembrane domain;
   C is a co-stimulatory signaling molecule;
   CD3ζ is a cytoplasmic signaling sequence derived from CD3ζ;
   esach "-" independently indicates a linking peptide or peptide bond connecting each of the above elements;
(ii) providing a DNT cell culture medium, the cell culture medium containing at least one DNT cell; transducing the expression vector of step (i) into the DNT cell, thereby obtaining a universal CAR-DNT cell targeting CD19 as described in the first aspect of the present invention; and
(iii) optionally detecting the universal CAR-DNT cell obtained in step (ii).

In another preferred example, the expression vector is selected from DNA, RNA, and a combination thereof.

In another preferred example, the expression vector is selected from the group consisting of: a plasmid, viral expression vector, transposon, and a combination thereof.

In another preferred embodiment, the viral expression vector is selected from the group consisting of: a lentiviral vector, adenoviral vector, retroviral vector, and a combination thereof.

In another preferred embodiment, the expression vector is a viral expression vector and before the method (ii), further comprising the step of: virally packaging the viral expression vector of step (i).

In another preferred embodiment, the viral expression vector is a retroviral expression vector, a lentiviral expression vector, preferably a lentiviral expression vector.

In a further preferred embodiment, the viral expression vector of step (i) is integrated with a polynucleotide sequence as shown in SEQ ID NO: 16.

In another preferred embodiment, in the step (ii), comprising co-transfecting the viral expression vector of step (i) with packaging plasmids psPAX2, pMD2.0G into a viral packaging a host cell.

In another preferred embodiment, the polynucleotide sequence in the step (i) contains a nucleotide sequence encoding a scFv of Formula (I) as shown in SEQ ID NO:15.

In another preferred example, in the step (i), the polynucleotide sequence contains a nucleotide sequence encoding the L in Formula (I) as shown in SEQ ID NO:8.

In another preferred example, in the step (i), the polynucleotide sequence contains a nucleotide sequence encoding H of Formula (I) as shown in SEQ ID NO:9.

In another preferred embodiment, in the step (i), the polynucleotide sequence contains a nucleotide sequence encoding the TM in Formula (I) as shown in SEQ ID NO: 10.

In another preferred embodiment, in the step (i), the polynucleotide sequence contains a nucleotide sequence encoding a C of Formula (I) as shown in SEQ ID NO: 11.

In another preferred embodiment, in the step (i), the polynucleotide sequence contains a nucleotide sequence encoding CD3ζ of Formula (I) as shown in SEQ ID NO: 12.

In another preferred example, the host cell packaged by virus is a mammalian cell, preferably a 293T cell.

In a further preferred embodiment, before the step (ii), further comprising the steps of:
(iia) collecting a peripheral blood sample from a healthy donor;
(iib) removing a CD4⁺CD8⁺ T cell from the peripheral blood sample, thereby obtaining a DNT cell;
(iic) culturing and expanding the DNT cell obtained in step (iib) in a culture flask coated with a CD3 monoclonal antibody with a suitable medium, thereby obtaining the DNT cell-containing culture system required in step (ii).

In another preferred example, the medium of the step (iic) is supplemented with a substance selected from the group consisting of: gentamicin, recombinant human interleukin 2, recombinant human interleukin 7, recombinant human interleukin 12, recombinant human interleukin 15, autologous plasma, AB serum, and a combination thereof.

In another preferred example, the medium of the step (iic) does not contain recombinant or wild-type human interleukin 4.

In another preferred embodiment, the medium of the step (iic) does not contain AB serum.

In another preferred example, in the medium of the step (iic), the concentration of the gentamicin is 40-80 units/ml (preferably 60 units/ml), the concentration of the recombinant human interleukin 2 is 150-1000 IU/ml (preferably 200-250 IU/ml), the concentration of the recombinant human interleukin 15 is 5-20 ng/ml (preferably 10 ng/ml), the concentration of the recombinant human interleukin 7 is 1-5 ng/ml (preferably 2 ng/ml), the concentration of the recombinant human interleukin 12 is 5-20 ng/ml (preferably 10 ng/ml), the concentration of the autologous plasma is 3-25 v% (preferably 10-20 v%), and/or the concentration of the AB serum is 4-8 v% (preferably 6 v%).

In another preferred example, in the step (ii), the concentration of the DNT cell in the DNT cell culture medium is from 1 × 10⁶ cells/ml to 4 × 10⁶ cells/ml, preferably from 1 × 10⁶ cells/ml to 2 × 10⁶ cells/ml, more preferably from 0.5 × 10⁶ cells/ml to 1 × 10⁶ cells/ml.

In another preferred embodiment, in the step (ii), the virus has an MOI of 0.1 to 10, preferably 0.1 to 8, preferably 5.

In a further preferred example, the detection of the step (iii) is performed after days 7-14 after the transduction of step (ii), preferably after day 8 or day 13.

In a third aspect of the present invention, it provides a pharmaceutical composition comprising:
(a) a universal CAR-T cell targeting CD19 as described in the first aspect of the present invention; and
(b) a pharmaceutically acceptable carrier, diluent or excipient.

In another preferred example, the pharmaceutical composition is a pharmaceutical composition in liquid form.

In a further preferred example, the pharmaceutical composition is an injection.

In another preferred example, the concentration of the universal CAR-T cells targeting CD 19 in the pharmaceutical composition is 1×10⁶-5×10⁶ cells/ml, preferably 1×10⁶-2×10⁶ cells/ml.

In a fourth aspect of the present invention, it provides a use of a universal CAR-T cell targeting CD19 as described in a first aspect of the present invention for the preparation of a pharmaceutical composition or preparation for the prevention and/or treatment of cancer.

In another preferred example, the tumor is selected from the group consisting of: hematologic neoplasm, solid tumor, and a combination thereof.

In a further preferred embodiment, the tumor is a hematologic neoplasm.

In another preferred embodiment, the hematologic neoplasm is selected from the group consisting of: acute myelocytic leukemia (AML), multiple myeloma (MM), chronic lymphocytic leukemia (CLL), acute lymphoid leukemia (ALL), diffuse large B-cell lymphoma (DLBCL), and a combination thereof.

In another preferred example, the solid tumor is selected from the group consisting of: gastric cancer, peritoneal metastasis from gastric cancer, liver cancer, leukemia, kidney tumor, lung cancer, carcinoma of small intestine, bone cancer, prostate cancer, colorectal cancer, breast cancer, colorectal cancer, cervical cancer, ovarian cancer, lymphoma, nasopharynx cancer, adrenal tumor, bladder tumor, non-small cell lung cancer (NSCLC), glioma, endometrial cancer, lung squamous carcinoma, anal carcinoma, head and neck neoplasm and a combination thereof.

In a fifth aspect of the present invention, it provides a method of preventing and/or treating a disease comprising the step of: administering to a subject in need a universal CAR-T cell targeting CD19 as described in a first aspect of the present invention, or a pharmaceutical composition as described in a third aspect of the present invention.

In another preferred embodiment, the disease is a cancer or tumor.

In another preferred example, the subject in need is a human or non-human mammal.

It should be understood that, within the scope of the present invention, each technical feature of the present invention described above and in the following (as examples) may be combined with each other to form a new or preferred technical solution, which is not listed here due to space limitations.

### Description of Figure

Figure 1 shows the structure of the three generations of CART.
Figure 2 shows the schematic structure of the humanized CD19 CAR construct.
Figure 3 shows a three-dimensional model of humanized CD 19 with mutations in the CDR1 domain (V27G).
Figure 4 shows the percentage of DNT cells in the culture system identified by CD3, CD4, CD8 antibody labeling using flow cytometry on day 8 and day 13 of culture, and the detection of efficiency of DNT cells infected by antibody-carrying lentivirus by specific anti-human CD19 variable region antibody labeling, from which the percentage of CART cells in the cell product is calculated.
Figure 5 shows the comparison of in vitro specific tumor killing activity of humanized CAR19-DNT against Hela and Hela-CD19 target cells on day 8 and 13 of culture, respectively; wherein 5-A shows real-time killing monitored by RTCA (Real-Time Cytotoxicity Assay) at an effector-to-target ratio of 2:1; 5-B shows real-time quantitative killing monitored by RTCA (Real-Time Cytotoxicity Assay) after 18 h of addition of effector cells at an effector-target ratio of 2:1.

### Detailed Description

After extensive and thorough research and extensive screening, the present inventors have developed a method for constructing universal antigen chimeric receptor CAR19-DNT cells for the first time. It is demonstrated that the universal antigen chimeric receptor CAR19-DNT cells provided by the present invention are derived from the peripheral blood of healthy donors, do not require complex gene editing methods to knock out the TCR gene that causes graft-versus-host disease, have low immunogenicity (do not cause host-versus-graft immune response and long retention time in the body), have high safety, specifically target human CD19 antigen, and have the advantage of significant tumor-killing effect, significant killing effect, and the construction method provided by the present invention can be produced on a large scale with low production cost. The present invention is completed on this basis.

### Terms

In order that the present disclosure may be more readily understood, certain terms are first defined. As used in this application, unless otherwise expressly provided herein, each of the following terms shall have the meaning given below.

As used herein, the term "about" may refer to a value or composition that is within an acceptable margin of error for a particular value or composition as determined by one of ordinary skill in the art, which will depend in part on how the value or composition is measured or determined.

As used herein, the terms "administer", "administering" are used interchangeably and refer to the physical introduction of the product of the present invention to a subject using any of a variety of methods and delivery systems known to those of skill in the art, including intravenous, intramuscular, subcutaneous, intraperitoneal, or spinal or other parenteral routes of drug delivery, such as by injection or infusion.

As used herein, the term "DNT cells" refers to Double Negative T cells, which are phenotypically distinct TCRαβ⁺ and/or TCRγδ⁺ T lymphocytes that express CD3 molecules but not CD4, CD8, NK, iNKT, and other characteristic surface molecules of cells (markers) playing a unique and diverse physiological role in the human immune system.

Double Negative T (DNT) cells are a subpopulation of CD3⁺CD4⁻CD8⁻ mature T lymphocytes normally present in peripheral blood, accounting for about 1-3% of peripheral blood mononuclear cells. DNT cells express CD3 molecules and αβ- or γδ-T Cell Receptor (TCR) on their surface, but do not express CD4 and CD8 molecules, and does not respond to constant natural killer T (invariant Nature Killer T, iNKT) cell-specific αGalCer, thus differing from conventional T cells, NK cells and NKT cells. DNT cells exert cytotoxic and antigen-presenting functions through a variety of natural human mechanisms while releasing cytokines/chemokines and activating a broader immune response, making it a promising clinical treatment candidate for a wide range of applications.

The advantages of DNT cells as cellular immunotherapy candidates include:
DNT cells can be used in a variety of tumor indications, it selectively targets the corresponding ligands expressed on various blood or solid tumors such as AML, lymphoma, cervical cancer and lung cancer through cell surface receptors such as NKG2D and DNAM-1, and secretes TNF-α, IFN-γ, Grazyme B, Peforin, IL-2 and IL-4 to exert direct and indirect tumor-killing activities. It can also be used in combination with immune checkpoint inhibitors such as PD-1, CTLA-4 and chemotherapeutic agents to have synergistic therapeutic effects.

Tumor cell killing by DNT cells is not restricted by MHC (histocompatibility complex) molecules, and allogeneic DNT cells donated by healthy donors are infused into patients without killing toxicity to normal cells in the patient's body and without affecting the further differentiation of hematopoietic stem cells, and do not cause Graft versus Host Disease (GvHD). There is also no Host versus Graft (HAVG) response, allowing the imported allogeneic DNT cells to remain in the patient's body for a longer period of time to exert tumor-killing activity.

DNT cells from healthy donors can be efficiently expanded to more than 5×10⁸ orders of magnitude per ml of peripheral blood and can be collected and frozen for multiple patient treatment, allowing clinical patients to be treated as soon as the diagnosis is clear, eliminating the waiting period for product preparation required for autologous CART products and making it a truly off-the-shelf universal (ready-to-use) cell product. Combining the above-mentioned broad-spectrum antitumor, high safety, versatility, and combination with other immunotherapies that DNA cells have, the present invention is designed to construct a universal humanized antigen chimeric receptor CART-19 product targeting CD19, and to develop a series of universal CART cell products based on the DNT cell platform without gene editing by derivation.

### Chimeric antigen receptors (CARs)

Chimeric Antigen Receptors (CARs) consist of an extracellular antigen recognition domain, usually a scFv (single-chain variable Fragment), a transmembrane region, and an intracellular co-stimulatory signaling domain. The design of CARs goes through the following process: first-generation CARs have only one intracellular signaling component, CD3ζ or FcyRI molecules, and because there is only one intracellular activation domain, it can only cause transient T cell proliferation and less cytokine secretion, but does not provide prolonged T cell proliferation signal and sustained in vivo anti-tumor effect, so that it does not achieve good clinical efficacy. The second generation CARs introduce a co-stimulatory molecule, such as CD28, 4-1BB, OX40 and ICOS, on the basis of the original structure, which has greatly improved the function compared with the first generation CARs, further enhancing the sustainability of CAR-T cells and their ability to kill tumor cells. Based on the second generation CARs, some new immune co-stimulatory molecules such as CD27 and CD134 are in tandem to develop into third and fourth generation CARs.

The extracellular segment of CARs recognizes one or more specific antigenic determinant (epitopes) and subsequently transduces this signal through intracellular domains, causing cell activation and proliferation, cytolytic toxicity and secretion of cytokines, which in turn removes target cells. PBMC from the patient's autologous or allogeneic (healthy donor) are first isolated, activated and genetically modified to generate immune cells for CAR, and subsequently injected into the patient which specifically kills tumor cells by directly recognizing their surface antigens in a non-MHC-restricted manner.

Specifically, the chimeric antigen receptor (CAR) of the present invention includes an extracellular domain, a transmembrane domain, and an intracellular domain. The extracellular domain includes a target-specific binding element (also referred to as an antigen-binding domain). Intracellular domains include co-stimulatory signaling regions and/or ζ-chain portions. The co-stimulatory signaling region refers to the portion of the intracellular domain that includes co-stimulatory molecules. Co-stimulatory molecules are cell surface molecules required for an effective response of lymphocytes to antigens and are not antigen receptors or their ligands.

A junction may be incorporated between the extracellular and transmembrane domains of the CAR, or between the cytoplasmic and transmembrane domains of the CAR.

As used herein, the terms "junction" and "hinge region" are used interchangeably and generally refer to any oligopeptide or polypeptide that serves to link the transmembrane domain to the extracellular or cytoplasmic domains of the polypeptide chain. The junction may comprise 0 to 300 amino acids, preferably 2 to 100 amino acids and most preferably 3 to 50 amino acids.

The CAR of the present invention, when expressed in immune cells, is capable of antigen recognition based on antigen binding specificity. When it binds antigens associated on tumor cells, it leads to tumor cell death and reduction or elimination of the patient's tumor load. The antigen-binding domain is preferably fused to an intracellular domain derived from one or more of the co-stimulatory molecules and/or the ζ chain. Preferably, the antigen-binding domain is fused to an intracellular domain from a combination of the CD28 costimulatory signaling molecule, the 4-1BB costimulatory signaling molecule, and the CD3ζ signaling domain.

As used herein, the basic structure of the chimeric antigen receptor of the present invention includes: a tumor-associated antigen binding region, an extracellular hinge region, a transmembrane region, and an intracellular signaling region. The selection of tumor-associated antigen directly affects its therapeutic effect on tumors. In the present invention, the chimeric antigen receptor of the present invention targets CD19.

The CD19 molecule is mainly expressed in early B cells and is a transmembrane glycoprotein specific to a B cell line of about 95 KDa. CD19 is expressed in both normal and malignant B lymphocytes and is considered to be the most reliable surface marker covering a long period of B cell development.

In a preferred embodiment of the present invention, a single chain antibody against CD19 is selected as the antigen-binding domain targeting CD19 in the CAR of the present invention.

In a preferred embodiment, the scFv has an amino acid sequence as shown in SEQ ID NO: 4 and can efficiently bind to CD19 molecules.

In the present invention, the antigen-binding domain targeting CD19 further comprises a conserved variant of the scFv, meaning that up to 10, preferably up to 8, more preferably up to 5, most preferably up to 3 amino acids are replaced by amino acids of close or similar nature to form a polypeptide compared to the amino acid sequence of the scFv of the present invention. In the present invention, the number of amino acids added, deleted, modified and/or substituted is preferably not more than 40%, more preferably not more than 35%, more preferably 1-33%, more preferably 5-30%, more preferably 10-25%, more preferably 15-20% of the total number of amino acids in the initial amino acid sequence. In the present invention, the number of the added, deleted, modified and/or substituted amino acids is typically 1, 2, 3, 4 or 5, preferably 1-3, more preferably 1-2, and most preferably 1.

For hinge regions and transmembrane regions (transmembrane domains), the CAR may be designed to include transmembrane domains fused to the extracellular domains of the CAR. In one embodiment, a transmembrane domain that is naturally associated with one of the domains in the CAR is used. In some examples, the transmembrane domain may be selected, or modified by amino acid substitution, to avoid binding such a domain to the transmembrane domain of the same or a different surface membrane protein, thereby minimizing interactions with other members of the receptor complex.

Preferably, the CAR constructs of the present invention have the following structure: CD8 leader sequence - CD19 scFv (VL-junction-VH) - CD8 hinge region - CD8 TM-41BB-CD3-ζ.

In a preferred embodiment, the amino acid sequence of the CAR of the present invention is shown at positions 1-489 of SEQ ID NO: 14. Of particular note, in the amino acid sequence of the CAR targeting CD 19 of the present invention, the position 173 corresponding to sequence SEQ ID NO: 14 is glycine (Gly).

### Universal CAR-T cells targeting CD19

As used herein, the terms "CAR19-DNT cell", "universal CAR-DNT cell targeting CD19", "CAR-T cell of the present invention", " universal CAR-T cell of the present invention" are used interchangeably, all refer to the universal double-negative T cells described in the first aspect of the present invention expressing a chimeric antigen receptor having a structure of Formula I that specifically targets a human CD19 molecule.

In the present invention, the universal CAR-DNT cells targeting CD19 express the CAR construct of the present invention.

Preferably, the TCR in the universal CAR-DNT cells targeting CD19 is not knocked out.

### Preparation methods

Also provided in the present invention is a method for preparing the universal CAR-DNT cells targeting CD19 of the present invention, comprising the steps of:
(i) providing an expression vector, the expression vector contains a nucleotide sequence expressing a CAR construct of the present invention.
(ii) providing a DNT cell culture medium, the cell culture medium contains at least one DNT cell; transducing the expression vector of step (i) into the DNT cell, thereby obtaining a universal CAR-DNT cell targeting CD19 of the present invention; and
(iii) optionally detecting the universal CAR-DNT cell obtained in step (ii).

Preferably, the expression vector in the method of preparation of the present invention is a viral expression vector, particularly preferably a lentiviral expression vector.

Notably, for the obtaining of the DNT cell culture medium in step (ii) of the described method, the following steps are required:
(iia) collecting a peripheral blood sample from a healthy donor;
(iib) removing a CD4⁺CD8⁺ T cell from the peripheral blood sample, thereby obtaining a DNT cell;
(iic) culturing and expanding the DNT cell obtained in step (iib) in a culture flask coated with a CD3 monoclonal antibody with a suitable medium, thereby obtaining the DNT cell-containing culture system required in step (ii).

Preferably, the medium of the step (iic) is supplemented with a substance selected from the group consisting of: gentamicin, recombinant human interleukin 2, recombinant human interleukin 7, recombinant human interleukin 12, recombinant human interleukin 15, autologous plasma, AB serum, and a combination thereof.

In another preferred example, in the medium of the step (iic), the concentration of the gentamicin is 40-80 units/ml (preferably 60 units/ml), the concentration of the recombinant human interleukin 2 is 150-1000 IU/ml (preferably 200-250 IU/ml), the concentration of the recombinant human interleukin 15 is 5-20 ng/ml (preferably 10 ng/ml), the concentration of the recombinant human interleukin 7 is 1-5 ng/ml (preferably 2 ng/ml), the concentration of the recombinant human interleukin 12 is 5-20 ng/ml (preferably 10 ng/ml), the concentration of the autologous plasma is 3-25 v% (preferably 10-20 v%), and/or the concentration of the AB serum is 4-8 v% (preferably 6 v%).

In a preferred embodiment of the present invention, the medium of the step (iic) does not contain recombinant or wild type human interleukin 4 and does not contain AB serum.

### Pharmaceutical compositions

The present invention provides a universal CAR-T cell targeting CD19 as described in the first aspect of the present invention, and a pharmaceutically acceptable carrier, diluent or excipient. In one embodiment, the pharmaceutical composition is a liquid formulation. Preferably, the formulation is an injection. Preferably, the concentration of the CAR-T cells in the formulation is 1×10⁵-1×10⁸ cells/ml, more preferably 1×10⁵-1×10⁶ cells/ml.

In one embodiment, the formulation may comprise a buffer such as neutral buffered saline, sulfate-buffered saline, etc.; a carbohydrate such as glucose, mannose, or glucan, mannitol; a protein; a polypeptide or amino acid such as glycine; an antioxidant; a chelating agent such as EDTA or glutathione; an adjuvant (e.g., aluminum hydroxide); and a preservative. The formulations of the present invention are preferably formulated for intravenous administration.

### Therapeutic applications

The present invention also provides a use of a universal CAR-T cell targeting CD19 of the first aspect of the present invention and a pharmaceutical composition described in the third aspect of the present invention for the preparation of pharmaceutical compositions or formulations for the prevention and/or treatment of cancer, and for the treatment/or prevention of cancer.

The cancers described include tumors of various progressive stages, including clinical stage I to IV tumors. Cancers may include non-solid tumors (such as hematologic neoplasm, e.g., leukemia and lymphoma) or solid tumors. Types of cancers treated with the CAR of the present invention include, but are not limited to, carcinomas, embryonal cell tumors and sarcomas, and certain leukemic or lymphoid malignancies, benign and malignant tumors, and malignant tumors such as sarcomas, carcinomas, and melanomas. It also includes adult tumors/cancers and pediatric tumors/cancers.

Hematologic cancers are cancers of the blood or bone marrow. Examples of hematologic (or hematogenous) cancers include leukemia, including acute leukemia (such as acute lymphoblastic leukemia, acute myelocytic leukemia, acute myelogenous leukemia and myeloblastic, promyelocytic, granule-monocyte type, monocytic leukemia and erythroleukemia), chronic leukemia (such as chronic myeloid (myelogenous) leukemia, chronic myelogenous leukemia and chronic lymphocytic leukemia), polycythemia vera, lymphoma, Hodgkin's disease, non-Hodgkin's lymphoma (painless and high-grade forms), multiple myeloma, Waldenström's macroglobulinemia, heavy chain disease, myelodysplastic syndrome, hairy cell leukemia and myelodysplasia.

Solid tumors are abnormal masses of tissue that do not usually contain cysts or areas of fluid. Solid tumors can be benign or malignant. The different types of solid tumors are named after the type of cells that form them (such as sarcomas, carcinomas and lymphomas). Examples of solid tumors such as sarcomas and carcinomas include fibrosarcoma, myxosarcoma, liposarcoma mesothelioma, lymphoid malignancies, pancreatic cancer and ovarian cancer.

The universal CAR-T cells of the present invention can also be used as a vaccine type for in vitro immunization and/or in vivo therapy in mammals. Preferably, the mammal is a human.

For ex vivo immune cell preparation, at least one of the following occurs in vitro prior to administration of the cells into the mammal: i) expansion of the cells, ii) introduction of the nucleic acid encoding the CAR into the cells, and/or iii) cryopreservation of the cells.

Isolated cell processing procedures are well known in the art and are discussed more fully below. Briefly, cells are isolated from a mammal (preferably human) and the cells are genetically modified (i.e., transduced or transfected in vitro) with a vector expressing the CARs disclosed herein. The CAR-modified cells may be administered to a mammalian recipient to provide therapeutic benefit. The mammalian recipient may be human, and the CAR-modified cells may be autologous relative to the recipient, or allogeneic, isogenic (syngeneic).

In addition to the use of cell-based vaccines with respect to isolated immune cells, the present invention also provides compositions and methods for use in vivo to enhance the immune response against targeted antigens in patients.

The present invention provides methods for treating tumors comprising administering to a subject in need thereof an effective amount of the universal CAR-T cells of the present invention.

The universal CAR-T cells of the present invention may be administered alone or as a pharmaceutical composition in combination with a diluent and/or with other components such as IL-2, IL-15, IL-17 or other cytokines or cell populations. Briefly, the pharmaceutical compositions of the present invention may comprise target cells as described herein, in combination with one or more pharmaceutically or clinically acceptable carriers, diluents or excipients. Such compositions may include buffers such as neutral buffered saline, sulfate-buffered saline, and the like; carbohydrates such as glucose, mannose, sucrose or glucan, mannitol; a protein; a polypeptide or amino acid such as glycine; an antioxidant; a chelating agent such as EDTA or glutathione; an adjuvant (e.g., aluminum hydroxide); and a preservative. The formulations of the present invention are preferably formulated for intravenous administration.

The pharmaceutical compositions of the present invention may be administered in a manner suitable for the disease to be treated (or prevented). The amount and frequency of administration will be determined by such factors as the characteristics of the patient's condition, the type and severity of the disease - although the appropriate dose may be determined by clinical trials.

When "immunologically effective amount," "antitumor effective amount," "tumor-inhibiting effective amount," or "therapeutic amount " is indicated, the precise amount of the composition of the present invention to be administered may be determined by a physician, taking into account individual differences in the age, weight, tumor size, degree of infection or metastasis, and disease of the patient (subject). It may be generally noted that pharmaceutical compositions comprising the T cells described herein may be administered at doses of 10⁴ to 10⁹ cells/kg body weight, preferably 10⁵ to 10⁷ cells/kg body weight (including all integer values in those ranges). T cell compositions may also be administered multiple times at these doses. Cells may be administered by using infusion techniques well known in immunotherapy (see e.g. Rosenberg et al., New Eng. J. of Med. 319:1676, 1988). The optimal dose and treatment regimen for a specific patient can be determined by monitoring the patient for signs of disease, and the treatment regimen is determined by a person skilled in the medical field.

Administration of the subject compositions may be performed in any convenient manner, including by spray method, injection, swallowing, infusion, implantation, or transplantation. The compositions described herein may be administered subcutaneously, intradermally, intratumorally, intra-nodally, intraspinal, intramuscularly, by intravenous (i.v.) injection or intraperitoneally, intrapleural to the patient. In another embodiment, the T-cell compositions of the present invention are preferably administered by i.v. intravenous injection. The composition of T cells may be injected directly into a tumor, lymph node, or location of infection (CART cell products are predominantly administered by intravenous infusion and may be employed for direct injection into tumors, lymph nodes, or infected sites).

In some embodiments of the present invention, the cells are activated and expanded to therapeutic levels using the methods described herein or other methods known in the art for expanding T cells to therapeutic levels, administered to the patient in combination with (e.g., before, at the same time, or after) any number of related forms of therapy, the forms of therapy include, but are not limited to, treatment with: the agents such as antiviral therapy, cidofovir, interleukin-2, IFN-γ, other cytotoxic chemotherapeutic agents such as cytarabine (also known as ARA-C), checkpoint inhibitors such as PD-1 antibodies, anti-CTLA-4 antibodies and drugs that inhibit cytokine storm, such as tocilizumab antibodies against IL-6 receptors, and other treatments. In further embodiments, the T cells of the invention can be used in combination with: chemotherapy, radiation, immunosuppressive agents such as cyclosporine, azathioprine, methotrexate, mycophenolate and antibodies or other immunotherapeutic agents. In further embodiments, the cellular compositions of the present invention are administered to a patient in combination with a bone marrow transplant, utilizing a chemotherapeutic agent such as fludarabine, external beam radiation therapy (XRT), cyclophosphamide (e.g., before, while, or after). For example, in one embodiment, the subject may undergo standard treatment with high-dose chemotherapy followed by peripheral blood stem cell transplantation. In some embodiments, after transplantation, the subject receives an infusion of the expanded immune cells of the present invention. In an additional embodiment, the expanded cells are administered before or after surgical procedure.

The dose of the above treatment administered to the patient will vary with the precise properties of the treated condition and the recipient of the treatment. The ratio of doses administered by a person may be implemented according to accepted practice in the art. Typically, 1 × 10⁶ to 1 × 10¹⁰ universal CAR-DNT cells of the present invention can be administered to a patient per treatment or per course of treatment, for example, by intravenous infusion.

### The main advantages of the present invention include:

1) The CD19 CAR of the present invention is a humanized chimeric antigen receptor, which has the advantage of being less immunogenic and less likely to cause immune rejection than an all-mouse primary chimeric antigen receptor.
2) CAR19-DNT cells in the present invention are universal immune cell products prepared without gene editing, with simple process and better safety, DNT cells do not require the participation of TCR molecules for tumor killing, are not MHC-restricted, and do not cause GvHD and HvG, and can be used as a universal immune cell product, avoiding the tedious construction process required for knocking out TCR and other genes process and the high efficiency of large-scale sequencing and off-targeting.
3) The method of constructing and expanding CAR-DNT provided in the present invention can achieve large-scale production and reduce production cost. Compared with 1-to-1 individualized therapy, this universal CAR-DNT cell product greatly reduces production cost, reduces the burden on patients.
4) The CAR-DNT cells provided in the present invention are not limited by the source of patients, and can expand in a large scale from immune cells of healthy donors, with better cell activity and immediate treatment for patients, solving the problems of low quantity, poor quality and insufficient quantity of lymphocytes in patients with mid- to late-stage tumors in the current autologous CART cell therapy process.

The present invention is further described below in connection with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: A Laboratory Manual (New York: Cold Spring Harbor Laboratory Press, 1989), or as recommended by the manufacturer. Percentages and parts are percentages by weight and parts by weight unless otherwise stated.

The present invention uses a second generation CD19-CAR containing humanized CD19 scFv (clone 11) with mutation V27G in the CDR1 region of VH, and FIG. 2 shows a schematic structure of the humanized CD19 CAR construct. The present invention is further described below by way of embodiments, but the invention is not limited to these specific embodiments.

### Example 1: Construction of Humanized Anti -CD19 FMC63 scFv-41BB-CD3ζ

The present invention inserts a humanized CD19-ScFv-CAR structure between the XbaI and EcoRI sites of the lentiviral vector, which contains an insertion fragment of humanized CD19 ScFv-41BB-CD3ζ between the XbaI and EcoRI cloning sites.

### 1.1 Humanized CD19 antibodies: sequences of VH and VL and scFv

The present invention obtained humanized CD19 scFv from a mouse CD19 FMC63 scFv clone, selected to mutate its CDR1 to humanized scFv (clone 11). The structure of humanized CD 19 scFv is: VL-linker peptide-VH. The sequence of the linker peptide is GSTSGSGKPGSGEGSTKG (SEQ ID NO.:7).

The nucleotide sequence in bold is the sequence of humanized CD19 VL (SEQ ID NO: 1); the nucleotide sequence of VH in normal font (SEQ ID NO: 2, with the mutation encoding G (ggc) marked in bold); the middle italic is (SEQ ID NO: 3) the nucleotide sequence (SEQ ID NO: 7) encoding the linker peptide sequence GSTSGSGKGPGSGEGSTKG.

Humanized CD19 scFv (SEQ ID NO: 4): bold for VL, normal font for VH, underlined for CDR region, the amino acid position of the mutation is position 27 of VH (SEQ ID NO: 6), shown as large italics G (V27G of VH)

The amino acid sequence in bold is the amino acid sequence of VL (SEQ ID NO: 5); the amino acid sequence of VH (SEQ ID NO: 6) in normal font; and the amino acid sequence of the linker peptide (SEQ ID NO: 7) in italics.

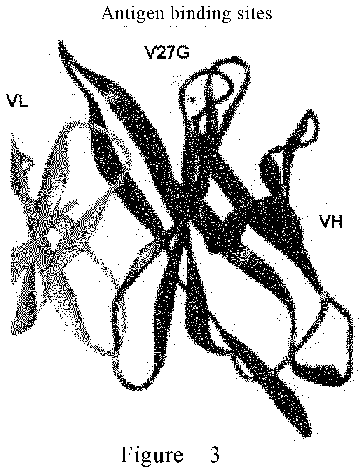

The present invention shows that mutation of the CDR1 region of the humanized CD19 antibody VH is found to improve the binding ability of the antibody to CD19 antigen by 3D modeling (Figure 3) and antigen-antibody binding experiments. 
DIQMTQSPSSLSASVGDRVTITCRASQDISKYLNWYQQKPGKAPKLLIYH
TSRLHSGVPSRFSGSGSGTDFTLTISSLQPEDFATYYCQQGNTLPYTFGG
GTKVEIKGSTSGSGKPGSGEGSTKGQVQLQESGPGLVKPSETLSLTCTVSGGS
LPDYGVSWIRQPPGKGLEWIGVIWGSETTYYNSALKSRVTISVDTSKNQFSL
KLSSVTAADTAVYYCAKHYYYGGSYAMDYWGQGTLVTVSS
 (SEQ ID NO.: 4)

### 1.2 Humanized CD19-CAR sequence

The structure of humanized CD19-CAR is shown in Figure 2. The humanized scFv CAR sequence was cloned using a lentiviral vector with EFla promoter.

The following nucleotide sequence is the CD8 leader sequence of humanized CD19ScFv-CD8 hinge-TM8-41BB-CD3ζ. The CAR structure includes human CD8 signal peptide, humanized CD19 scFv (VL-hinge-VH), CD8 hinge, CD8 transmembrane, 41BB co-stimulatory domain and CD3ζ activation domain (Figure 2).

CD8 leader sequence-CD19 scFv (V_{L}-Linker-V_{H})-CD8 hinge-CD8 TM-41BB-CD3-zeta:
<CD8 leader> 
ATGGCCTTACCAGTGACCGCCTTGCTCCTGCCGCTGGCCTTGCTGCTCCACGCCGCC
AGGCCG (SEQ ID NO.: 8)
<humanized CD19 (VL-linker-VH), clone 11 scFv> (bold marker is the mutation sequence ggc encoding V27G) 
gatattcagatgacccagagcccgagcagcctgagcgcgagcgtgggcgatcgcgtgacc
attacctgccgcgcgagccaggatattagcaaatatctgaactggtatcagcagaaaccg
ggcaaagcgccgaaactgctgatttatcataccagccgcctgcatagcggcgtgccgagc
cgctttagcggcagcggcagcggcaccgattttaccctgaccattagcagcctgcagccg
gaagattttgcgacctattattgccagcagggcaacaccctgccgtatacctttggcggc
ggcaccaaagtggaaattaaa
ggctccacctctggatccggcaagcccggatctggcgagggatccaccaagggc
caggtgcagctgcaggaaagcggcccgggcctggtgaaaccgagcgaaaccctgagcctg
acctgcaccgtgagcggcggcagcctgccggattatggcgtgagctggattcgccagccg
ccgggcaaaggcctggaatggattggcgtgatttggggcagcgaaaccacctattataac
agcgcgctgaaaagccgcgtgaccattagcgtggataccagcaaaaaccagtttagcctg
aaactgagcagcgtgaccgcggcggataccgcggtgtattattgcgcgaaacattattat
tatggcggcagctatgcgatggattattggggccagggcaccctggtgaccgtgagcagc
(SEQ ID NO.: 15)
<CD8 Hinge> 
ACCACGACGCCAGCGCCGCGACCACCAACACCGGCGCCCACCATCGCGTCGCAGCC
CCTGTCCCTGCGCCCAGAGGCGTGCCGGCCAGCGGCGGGGGGCGCAGTGCACACGA
GGGGGCTGGACTTCGCCTGTGAT (SEQ ID NO.: 9)
<CD8 TM> 
ATCTACATCTGGGCGCCCCTGGCCGGGACTTGTGGGGTCCTTCTCCTGTCACTGGTT
ATCACCCTTTACTGC (SEQ ID NO.: 10)
<4-1BB Co-stimulated domains > 
AAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAGAA
GGAGGATGTGAACTG (SEQ ID NO.: 11)
<CD3 ζ> 
AGAGTGAAGTTCAGCAGGAGCGCAGACGCCCCCGCGTACAAGCAGGGCCAGAACC
AGCTCTATAACGAGCTCAATCTAGGACGAAGAGAGGAGTACGATGTTTTGGACAAG
AGACGTGGCCGGGACCCTGAGATGGGGGGAAAGCCGAGAAGGAAGAACCCTCAGG
AAGGCCTGTACAATGAACTGCAGAAAGATAAGATGGCGGAGGCCTACAGTGAGAT
TGGGATGAAAGGCGAGCGCCGGAGGGGCAAGGGGCACGATGGCCTTTACCAGGGT
CTCAGTACAGCCACCAAGGACACCTACGACGCCCTTCACATGCAGGCCCTGCCCCC
TCGCTAATAG (SEQ ID NO.: 12)
<EcoRI restriction site>
   gaattc (SEQ ID NO.: 13)

The amino acid sequence of the humanized CD19-4-1BB-CD3-CAR protein is as follows (for the construct structure, see Figure 3), wherein mutations in CDR1 VH are identified by bold underlines:

MALPVTALLLPLALLLHAARPDIQMTQSPSSLSASVGDRVTITCR
ASQDISKYLNWYQQKPGKAPKLLIYHTSRLHSGVPSRFSGSGSGT
DFTLTISSLQPEDFATYYCQQGNTLPYTFGGGTKVEIKGSTSGSG
KPGSGEGSTKGQVQLQESGPGLVKPSETLSLTCTVSGGSLPDYG
VSWIRQPPGKGLEWIGVIWGSETTYYNSALKSRVTISVDTSKNQF
SLKLSSVTAADTAVYYCAKHYYYGGSYAMDYWGQGTLVTVSST
TTPAPRPPTPAPTIASQPLSLRPEACRPAAGGAVHTRGLDFACDI
YIWAPLAGTCGVLLLSLVITLYCKRGRKKLLYIFKQPFMRPVQTT
QEEDGCSCRFPEEEEGGCELRVKFSRSADAPAYKQGQNQLYNEL
NLGRREEYDVLDKRRGRDPEMGGKPRRKNPQEGLYNELQKDKM
AEAYSEIGMKGERRRGKGHDGLYQGLSTATKDTYDALHMQALP
PR (SEQ ID NO.: 14)

The nucleotide sequence of humanized CD19-4-1BB-CD3-CAR protein is as follows:

ATGGCCTTACCAGTGACCGCCTTGCTCCTGCCGCTGGCCTTGCTGCTCCACGCCGCC
AGGCCG
gatattcagatgacccagagcccgagcagcctgagcgcgagcgtgggcgatcgcgtgacc
attacctgccgcgcgagccaggatattagcaaatatctgaactggtatcagcagaaaccg
ggcaaagcgccgaaactgctgatttatcataccagccgcctgcatagcggcgtgccgagc
cgctttagcggcagcggcagcggcaccgattttaccctgaccattagcagcctgcagccg
gaagattttgcgacctattattgccagcagggcaacaccctgccgtatacctttggcggc
ggcaccaaagtggaaattaaa
ggctccacctctggatccggcaagcccggatctggcgagggatccaccaagggc
caggtgcagctgcaggaaagcggcccgggcctggtgaaaccgagcgaaaccctgagcctg
acctgcaccgtgagcggcggcagcctgccggattatggcgtgagctggattcgccagccg
ccgggcaaaggcctggaatggattggcgtgatttggggcagcgaaaccacctattataac
agcgcgctgaaaagccgcgtgaccattagcgtggataccagcaaaaaccagtttagcctg
aaactgagcagcgtgaccgcggcggataccgcggtgtattattgcgcgaaacattattat
tatggcggcagctatgcgatggattattggggccagggcaccctggtgaccgtgagcagc
ACCACGACGCCAGCGCCGCGACCACCAACACCGGCGCCCACCATCGCGTCGCAGCC
CCTGTCCCTGCGCCCAGAGGCGTGCCGGCCAGCGGCGGGGGGCGCAGTGCACACGA
GGGGGCTGGACTTCGCCTGTGAT
ATCTACATCTGGGCGCCCCTGGCCGGGACTTGTGGGGTCCTTCTCCTGTCACTGGTT
ATCACCCTTTACTGC
AAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAGAA
GGAGGATGTGAACTG
AGAGTGAAGTTCAGCAGGAGCGCAGACGCCCCCGCGTACAAGCAGGGCCAGAACC

AGCTCTATAACGAGCTCAATCTAGGACGAAGAGAGGAGTACGATGTTTTGGACAAG
AGACGTGGCCGGGACCCTGAGATGGGGGGAAAGCCGAGAAGGAAGAACCCTCAGG
AAGGCCTGTACAATGAACTGCAGAAAGATAAGATGGCGGAGGCCTACAGTGAGAT
TGGGATGAAAGGCGAGCGCCGGAGGGGCAAGGGGCACGATGGCCTTTACCAGGGT
CTCAGTACAGCCACCAAGGACACCTACGACGCCCTTCACATGCAGGCCCTGCCCCC
TCGCTAATAG (SEQ ID NO.: 16)

### Example 2: Lentiviral packaging

293T cells were cultured at 37°C in a 5% CO₂ incubator with DMEM + 10% FBS. on day 2, when the cells reached 90% confluence, the expression plasmids and packaging plasmids psPAX2 and pMD2.0G were used to co-transform, plasmids mixed in suitable molar ratios were added to the culture vessels, gently shaken, mixed, and placed in the incubator. After 48-72 h, the virus can be harvested. After centrifugation to remove floating dead 293T cells, the virus-containing medium is then filtered, concentrated, purified, dispensed, frozen at -80°C and titers are measured.

### Example 3: Preparation of CAR-19DNT cells

### 3.1 Collection of human peripheral blood samples

Collect 30-400 ml of peripheral blood from a healthy donor into heparin-containing sodium tube.

### 3.2 Preparation and assay of CAR19-DNT cells

### 3.2.1 Preparation of CAR19-DNT cells

### Method I:

On day 0, removal of CD4⁺, CD8⁺ T cells by Resetting with RBC using Rossettsep^{®} kit (Stem Cell Technologies Inc) according to the manufacturer's instructions. Blood samples were incubated with anti-human CD4 and CD8 removal reagent markers for 20 minutes at room temperature, and then blood was stratified in 50 ml centrifuge tubes with equal volumes of Ficoll-Hypaque in a density gradient. After centrifugation at 2500 rpm for 25 min, collection of CD4⁺ and CD8⁺ cells at the interface of Ficoll and plasma that have been removed from PBMC, i.e. DNT cells, washed once with 0.9% physiological saline. The obtained DNT cells were cultured at 1-6×10⁶ cells/ml in 75 cm² culture flasks at 37°C and 5% CO₂ in AIM-V medium, said flasks having been coated with anti-human CD3 monoclonal antibody (clone OKT3) (5-20 µg/ml), AIM-V medium containing gentamicin (60 units/ml), recombinant human interleukin 2 (250 IU/ml), recombinant human interleukin 15 (10 ng/ml), recombinant human interleukin 7 (2 ng/ml), recombinant human interleukin 12 (10 ng/ml), and autologous plasma (20 v%).

On day 1, the DNT cells obtained from the preparation were cultured overnight and then infected overnight by adding lentivirus with an MOI of 5, followed by supplementation of AIM-V medium daily or every other day depending on the cell status;

On day 5, DNT cells were fully activated and proliferated vigorously, and DNT cells were adjusted to 1-3 ×10⁶ cells/ml concentration with fresh AIM-V proliferation medium (containing 60 units/ml gentamicin, 500 IU/ml recombinant human interleukin 2 and recombinant human interleukin 15 (10 ng/ml), recombinant human interleukin 7 (2 ng/ml), recombinant human interleukin 12 (10 ng/ml), and autologous plasma (10 v%) ) to continue the expansion culture;

On day 7, pass to 175cm² culture flask and continue to culture for 3 days at 1-3 ×10⁶ cells/ml in AIM-V medium, said AIM-V medium contains gentamicin (60 units/ml), recombinant human interleukin 2 (250IU/ml), recombinant human interleukin 15 (10ng/ml), recombinant human interleukin 12 (10ng/ml) and 50ng/ml anti-human CD3 monoclonal antibody.

On day 10, cells from 175 cm² culture flasks were passed into culture bags and continued to be cultured at 1-3 × 10⁶ cells/ml in AIM-V medium until day 14, said AIM-V medium containing gentamicin (60 units/ml), recombinant human interleukin 2 (500 IU/ml), recombinant human interleukin 15 (10ng/ml), recombinant human interleukin 12 (10ng/ml) and 100ng/ml anti-human CD3 monoclonal antibody.

On day 14, CAR-19 DNT cells were harvested and collected in 250 ml pointed-bottom centrifuge flasks, centrifuged at 900 × g for 10 min, and washed with saline containing 2.5% human albumin (also known as "solvent"). The collected CAR-19-DNT cells were frozen in liquid nitrogen at a concentration of 0.1-1×10⁷ cells/mL, and this is the finished CAR19-DNT cell preparation, which is available for clinical use after passing quality control.

### Method II:

On day 0, CD4⁺, CD8⁺ T cells were removed by Resetting with RBC using the Rossettsep^{®} kit (Stem Cell Technologies Inc) according to the manufacturer's instructions. Blood samples were incubated with anti-human CD4 and CD8 removal reagent markers for 20 minutes at room temperature, and then blood was stratified in 50 ml centrifuge tubes with equal volumes of Ficoll-Hypaque in a density gradient. After centrifugation at 2500 rpm for 25 min, collection of CD4⁺ and CD8⁺ cells at the interface of Ficoll and plasma that have been removed from PBMC, i.e. DNT cells, washed once with 0.9% physiological saline. The purified DNT cells (beads: DNT cells = 1:1) were mixed with CD3/CD28 beads and cultured in 75 cm² flasks at 1-6×10⁶ cells/ml in AIM-V medium at 37°C and 5% CO₂, AIM-V medium containing gentamicin (60 units/ml), recombinant human interleukin 2 (500 IU/ml), recombinant human interleukin 15 (2 ng/ml), recombinant human interleukin 7 (2 ng/ml), recombinant human interleukin 12 (10 ng/ml), and autologous plasma (20 v%).

On day 1, the DNT cells obtained from the preparation were cultured overnight and then infected overnight by adding lentivirus with an MOI of 5, followed by supplementation of AIM-V medium daily or every other day depending on the cell status;

On day 5, remove CD3/CD28 beads by blowing the DNT cells in the culture flask and passing them through the magnetic rack and DNT cells were adjusted to 1-3 × 10⁶ cells/ml concentration with fresh AIM-V proliferation medium (containing 60 units/ml gentamicin, 500 IU/ml recombinant human interleukin 2 and recombinant human interleukin 15 (10 ng/ml), recombinant human interleukin 7 (2 ng/ml), recombinant human interleukin 12 (10 ng/ml), and autologous plasma (10 v%) ) to continue the expansion culture;

On day 7, pass to 175cm² culture flask and continue to culture for 3 days at 1-3 ×10⁶ cells/ml in AIM-V medium, said AIM-V medium contains gentamicin (60 units/ml), recombinant human interleukin 2 (500IU/ml), recombinant human interleukin 15 (10ng/ml), recombinant human interleukin 12 (10ng/ml).

On day 10, cells from 175 cm² culture flasks were passed into culture bags and continued to be cultured at 1-3 × 10⁶ cells/ml in AIM-V medium until day 14, said AIM-V medium containing gentamicin (60 units/ml), recombinant human interleukin 2 (500 IU/ml), recombinant human interleukin 15 (10ng/ml), recombinant human interleukin 12 (10ng/ml).

On day 14, CAR-19 DNT cells were harvested and collected in 250 ml pointed-bottom centrifuge flasks, centrifuged at 900 × g for 10 min, and washed with saline containing 2.5% human albumin (also known as "solvent"). The collected CAR-19-DNT cells were frozen in liquid nitrogen at a concentration of 0.1-1×10⁷ cells/mL, and this is the finished CAR19-DNT cell preparation, which is available for clinical use after passing quality control.

### 3.2.2 CAR-19 DNT cell phenotype and positive rate detection

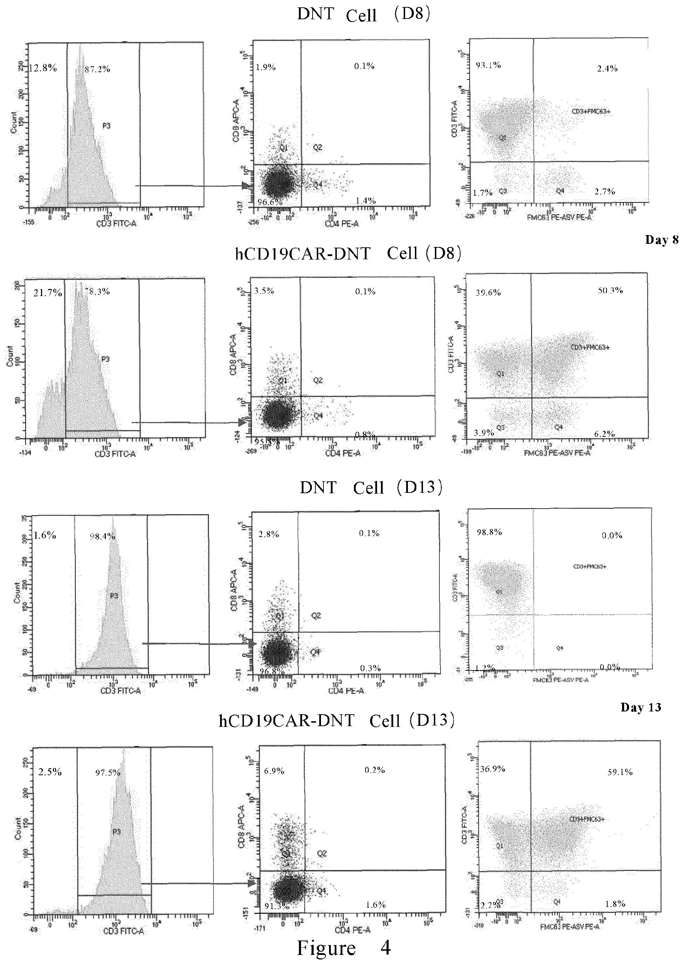

The DNT phenotype and CAR19-DNT positive rate were tested on day 8 and day 13, respectively, at this time, high purity DNT cells (up to 85% purity or more, as in Figure 4) as well as CAR19-DNT cells with high hCD19-CAR positive can be obtained. DNT cells in the culture system were identified by anti-human CD3 antibody, CD4 antibody, and CD8 antibody labeling using flow cytometry (Figure 4), while the transduction positive rate was detected by FMC63-CD19 antibody labeling with anti-humanized CD19 (Figure 4).

### Example 4: Cell killing assay

Hela cells expressing CD19 molecules (stable cell line of Hela-CD19) were established as target cells by lentiviral infection, and Hela cells not expressing CD19 were used as target cell control; DNT cells without transduced CAR-CD19 and CD19CAR-DNT cells with transduced humanized CAR-CD19 collected on day 8 and day 13 of culture, respectively, were used as effector cells with an effector cell-target ratio of 2:1, and killing was monitored in real time with RTCA (Real-Time Cytotoxicity Assay).

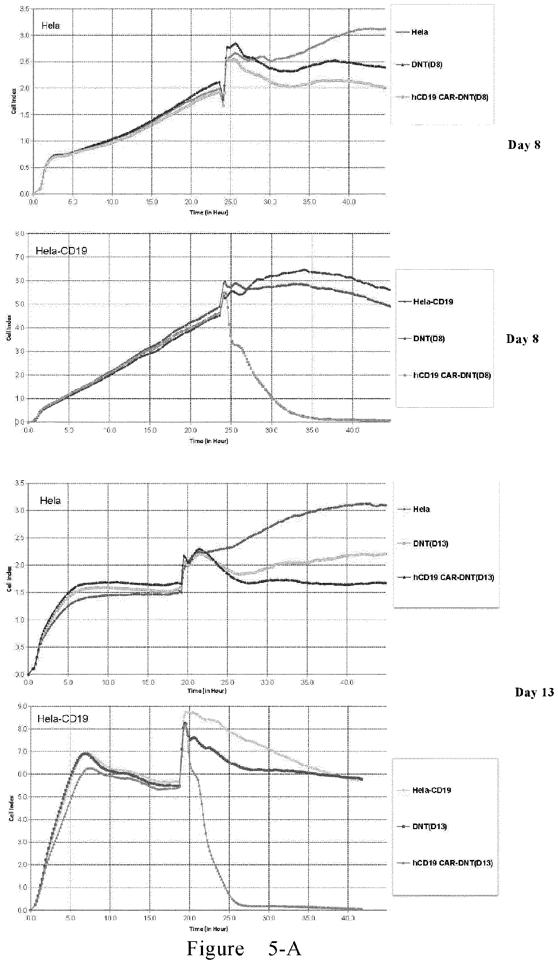

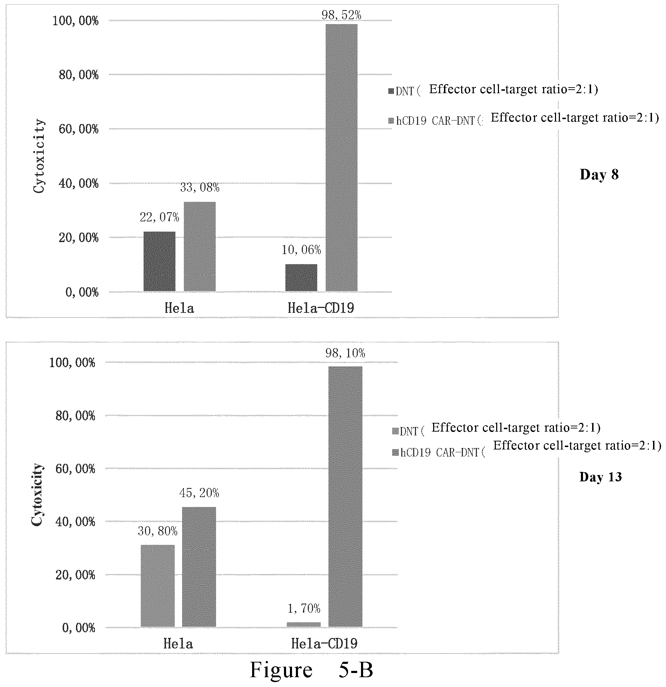

The results are shown in Figure 5: CAR19-DNT cells transduced with humanized CD19CAR specifically kill Hela-CD19 cells compared with DNT cells not transduced with CD19-CAR, and there is no significant difference between the above two cells in killing CD19-negative Hela cells. This suggests that humanized CAR19-DNT cells specifically target Hela-CD19 target cells expressing CD19 antigen (Figure 5). 5-A shows the real-time dynamic killing monitored by RTCA at an effector cell-target ratio of 2:1; the real-time quantitative killing results monitored by RTCA when the maximum killing time is reached (18 h) are shown in Figure 5-B. The quantitative killing results show that the specific killing of Hela-CD19 by CAR19-DNT cells on day 8 and 13 of culture is as high as 98.52% and 98.10%, respectively (Figure 5-B), and the maximum killing effect can be maintained continuously with the extension of the killing time (Figure 5-A).

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. A universal CAR-T cell targeting CD19, wherein the universal CAR-T cell expressing an exogenous CAR construct, the CAR construct having a structure as shown in Formula I,
L-scFv-H-TM-C-CD3ζ (Formula I)
wherein
L is none or a signal peptide sequence;
scFv is a sequence of the single-chain variable region of an antibody targeting CD 19;
H is none or a hinge region;
TM is a transmembrane domain;
C is a co-stimulatory signaling molecule;
CD3ζ is a cytoplasmic signaling sequence derived from CD3ζ;
esach "-" independently indicates a linking peptide or peptide bond connecting each of the above elements.

2. The universal CAR-T cell targeting CD19 of claim 1, wherein the universal CAR-T cell targeting CD19 is a universal CAR-double negative T cell targeting CD19 (CAR-DNT cell).

3. The universal CAR-T cell targeting CD19 of claim 1, wherein the TCR in the universal CAR-T cell is not knocked out.

4. The universal CAR-T cell targeting CD19 of claim 1, wherein the CAR construct has an amino acid sequence as shown in SEQ ID NO: 14.

5. A method for preparing a universal CAR-DNT cell targeting CD19 of claim 1, comprising the steps of:
(i) providing an expression vector, the expression vector containing a nucleotide sequence encoding a CAR construct as shown in Formula I;
L-scFv-H-TM-C-CD3ζ (Formula I)
wherein
L is none or a signal peptide sequence;
scFv is a sequence of the single-chain variable region of an antibody targeting CD 19;
H is none or a hinge region;
TM is a transmembrane domain;
C is a co-stimulatory signaling molecule;
CD3ζ is a cytoplasmic signaling sequence derived from CD3ζ;
esach "-" independently indicates a linking peptide or peptide bond connecting each of the above elements;
(ii) providing a DNT cell culture medium, the cell culture medium containing at least one DNT cell; transducing the expression vector of step (i) into the DNT cell, thereby obtaining a universal CAR-DNT cell targeting CD19 as described in the first aspect of the present invention; and
(iii) optionally detecting the universal CAR-DNT cell obtained in step (ii).

6. The method of claim 5, wherein the expression vector is a viral expression vector.

7. The method of claim 5, wherein the viral expression vector of step (i) is integrated with a polynucleotide sequence as shown in SEQ ID NO: 16.

8. A pharmaceutical composition comprising:
(a) a universal CAR-T cell targeting CD19 of claim 1 or 2; and
(b) a pharmaceutically acceptable carrier, diluent or excipient.

9. Use of a universal CAR-T cell targeting CD19 of claim 1 or 2 for the preparation of a pharmaceutical composition or preparation for the prevention and/or treatment of cancer.

10. The use of claim 9, wherein the tumor is selected from the group consisting of: hematologic neoplasm, solid tumor, and a combination thereof.
